# EUROPEAN PATENT APPLICATION

(11) **EP 4 659 697 A1**
(43) Date of publication of application: **10.12.2025**
(21) Application number: 25180815.0
(22) Date of filing: 04.06.2025
(51) Int. Cl.: A61B 34/30

(54) **LINEAR MEMBER MOVING DEVICE AND LINEAR MEMBER MOVING SYSTEM**

(30) Priority: 05.06.2024 KR 20240073685
(71) Applicant: Endorobotics Co., Ltd., Seoul 02841 (KR)
(72) Inventor: WON, Seong Hyeon, 02577 Seoul (KR); KIM, Chan Woo, 02577 Seoul (KR); LEE, Yong Jung, 02577 Seoul (KR); SHIN, Woo Cheol, 02577 Seoul (KR); KIM, Kyung Nam, 02577 Seoul (KR)
(74) Representative: Maiwald GmbH

(57) **Abstract**

A linear member moving device and a linear member moving system are disclosed. A linear member moving device according to one aspect of the present disclosure is a linear member moving device for moving a linear member in an extension direction of the linear member, and includes a main body, a pair of first grip members that is disposed to face each other with the linear member interposed therebetween and operatively supported by the main body to grip or release the grip of the linear member, a pair of second grip members that is disposed to face each other with the pair of first grip members interposed therebetween and that pressurizes or depressurizes the pair of first grip members so that the pair of first grip members grips or releases the grip of the linear member and operatively supported by the main body to move in the extension direction of the linear member, a first driving module that provides a driving force for the pair of second grip members to pressurize or depressurize the pair of first grip members and move in the extension direction of the linear member, and a second driving module that provides a driving force for the pair of first grip members to rotate about a central axis in the extension direction of the linear member.

## Description

### CROSS-REFERENCE TO RELATED APPLICATION

This application claims priority to and the benefit of Korean Patent Application No. 10-2024-0073685, filed on June 5, 2024, the disclosure of which is incorporated herein by reference in its entirety.

### BACKGROUND

### Field

The present disclosure relates to a linear member moving device and a linear member moving system.

### Description of the Related Art

As robots advance, efforts are being made to have robots, which were previously only used in industrial settings, replace human labor in service fields such as housework, cooking, and serving.

Recently, many procedures are being performed to insert surgical instruments in the form of tubes, such as catheters and guide wires, to treat arrhythmias or blood vessels.

In particular, robotic systems are widely used to shorten treatment times and perform precise procedures.

Conventionally, surgical instruments in the form of tubes, such as catheters and guide wires, were inserted by hand or by using insertion instruments with roller structures.

It varies depending on the procedure, but most procedures involve using multiple types of surgical instruments to insert and then remove the device multiple times.

Meanwhile, as a related art, US Patent No. 6096004 depicts a tubular surgical tool inserter.

The tubular surgical tool inserter of the related art performs a procedure by inserting a surgical tool using a roller.

In this way, when performing a procedure by inserting a surgical tool using a roller, the roller makes linear contact with the surgical tool, so excessive force is required to obtain the necessary frictional force, which not only causes damage to the surgical tool, but also makes it difficult to precisely insert the surgical tool.

Meanwhile, there is a need to rotate a tubular surgical tool during the surgical procedure. To solve this need, conventionally, the surgical tool was rotated by rotating the entire device that moves the surgical tool or by rubbing the surgical tool with a roller.

However, there are problems in that when the entire device is rotated, the device volume becomes excessively large and strong force is required, and in the case of the method of rubbing with a roller, there is a limit to the rotation angle, and it may not be applied to a linear automatic inserter.

### SUMMARY

The present disclosure is to solve the above problems, and an object of the present disclosure is to provide a linear member moving device that is configured to have a small volume and no limit to the rotation angle, which is capable of moving a linear member in the extension direction of the linear member as well as rotating the linear member about a central axis in an extension direction of the linear member.

Objects of the present disclosure are not limited to the objects mentioned above, and other objects not mentioned will be clearly understood by those skilled in the art to which the present disclosure belongs from the description below.

According to one aspect of the present disclosure, there is provided a linear member moving device that moves a linear member in an extension direction of the linear member, the linear member moving device including: a main body; a pair of first grip members that is disposed to face each other with the linear member interposed therebetween and operatively supported by the main body to grip or release the grip of the linear member; a pair of second grip members that is disposed to face each other with the pair of first grip members interposed therebetween and that pressurizes or depressurizes the pair of first grip members so that the pair of first grip members grips or releases the grip of the linear member and operatively supported by the main body to move in the extension direction of the linear member; a first driving module that provides a driving force for the pair of second grip members to pressurize or depressurize the pair of first grip members and move in the extension direction of the linear member; and a second driving module that provides a driving force for the pair of first grip members to rotate about a central axis in the extension direction of the linear member.

In this case, the pair of second grip members may be movable in the extension direction of the linear member together with the linear member and the pair of first grip members in a state of pressurizing the pair of first grip members, and the pair of first grip members may be rotatable about the central axis in the extension direction of the linear member together with the linear member in a state of being pressurizing by the pair of second grip members.

In this case, the pair of second grip members may be movable in the extension direction of the linear member in a state where the pair of first grip members is depressurized, and the pair of first grip members may be rotatable about the central axis in the extension direction of the linear member in a state of being depressurized by the pair of second grip members.

Meanwhile, the first driving module may include a gripper that operates the pair of second grip members to pressurize or depressurize the pair of first grip members, and the gripper may include a pair of pressing members that is respectively coupled with the pair of second grip members, and a gripper actuator that provides a driving force for the pair of second grip members to pressurize or depressurize the pair of first grip members to the pair of pressing members.

In this case, the first driving module may include an actuator that provides a driving force for moving the gripper in the extension direction of the linear member.

Meanwhile, the first driving module may include a pair of pressing members that is coupled with the pair of second grip member, a driving motor that provides a driving force for operating the pair of pressing members, and a pair of link units that operably connects the driving motor and the pair of pressing members, and moves the pair of pressing members along a first trajectory moving in one direction of the extension direction of the linear member and a second trajectory moving away from and toward the linear member while moving in the other direction of the extension direction of the linear member, and the linear member may be gripped when the pair of pressing members moves along the first trajectory, and the grip of the linear member may be released when the pair of pressing members moves along the second trajectory.

In this case, each link unit may include a driving link that has one end portion receiving the driving force of the driving motor and rotates about a third direction that is perpendicular to both a first direction, which is the extension direction of the linear member, and a second direction, which is the direction in which the pair of second grip members approaches and moves away from each other, a connecting link that has one end portion pivotally connected to the other end portion of the driving link and the other end portion coupled to each of the pressing members, and an auxiliary link that has one end portion pivotally connected to one side of the main body and the other end portion pivotally coupled to a central region of the connecting link, and the driving links of each link unit receive the driving force of the driving motor and rotate in directions opposite to each other.

In this case, the first driving module may further include a pair of link drive gears that is axial-coupled to rotate together with one end portion of the driving link of each link unit, the pair of link drive gears may be mutually meshed and rotate in directions opposite to each other, and one of the pair of link drive gears may be operably connected to the driving motor to provide a driving force.

Meanwhile, the second driving module may include a rotating member that is disposed to be spaced apart from the pair of first grip members in the extension direction of the linear member and is supported by the main body so as to rotate about the central axis in the extension direction of the linear member, a motor that is operably connected to the rotating member to rotate the rotating member, and a connecting member that interconnects the rotating member and the pair of first grip members, with at least one connecting member provided for each of the first grip members.

In this case, the connecting member may have a bar shape extending in the extension direction of the linear member, and a connecting member penetration hole through which the connecting member penetrates may be formed in each of the first grip members.

In this case, the connecting member may guide each of the first grip members when each of the first grip members moves in the extension direction of the linear member.

Meanwhile, the connecting member penetration hole may extend in a direction in which the pair of first grip members comes closer or move away from each other.

In this case, the connecting member may guide the pair of first grip members when the pair of first grip members performs a gripping or grip-releasing operation.

Meanwhile, each of the pair of first grip members may be formed so that at least a part of one surface facing the linear member is formed as a plane, and at least a part of the other surface located on a side opposite to the one surface is formed as a curved surface.

Meanwhile, an elastic body may be disposed between the pair of first grip members to provide an elastic force in a direction in which the pair of first grip members moves away from each other.

Meanwhile, each of the pair of second grip members may include a second grip member body spaced apart from the linear member in a direction perpendicular to the extension direction of the linear member, and at least one rolling member coupled to the second grip member body so as to make rolling motion by contacting the pair of first grip members when the pair of first grip members rotates in a state of gripping the linear member.

Meanwhile, the linear member moving device may further include a push unit that pushes the pair of first grip members in a movement direction of the second grip member when the pair of second grip members moves in the extension direction of the linear member in a state of completely depressurizing the pair of first grip members.

In this case, the push unit may be formed to move in the extension direction of the linear member by the driving force of the first driving module moving the pair of second grip members in the extension direction of the linear member.

In this case, the push unit may be supported by a part of the first driving module that moves in the extension direction of the linear member together with the pair of second grip member.

Meanwhile, the push unit may include a pair of push members spaced apart in the extension direction of the linear member with the pair of first grip members interposed therebetween.

In this case, each push member may include a rotary plate having a linear member penetration hole formed through which the linear member penetrates and disposed to face any one of both sides of the pair of first grip members in the extension direction of the linear member, and a push member body that supports the rotary plate so as to be rotatable about the linear member penetration hole.

Meanwhile, the linear member moving device may further include a force sensor interposed between both sides of the pair of first grip members in the extension direction of the linear member and the pair of push members.

Meanwhile, according to another aspect of the present disclosure, there is provided a linear member moving system including a pair of linear member moving devices, in which the pair of linear member moving devices includes a first linear member moving device and a second linear member moving device, and when a first driving module of the first linear member moving device provides a driving force for moving in one direction of extension directions of the linear member in a state where a pair of second grip members of the first linear member moving device pressurizes a pair of first grip members of the first linear member moving device, the first driving module of the second linear member moving device provides a driving force for moving in the other direction of the extension directions of the linear member in a state where a pair of second grip members of the second linear member moving device depressurizes a pair of first grip members of the second linear member moving device.

According to the above configuration, the linear member moving device according to one aspect of the present disclosure performs separately or simultaneously the operation of linearly moving the linear member in the extension direction of the linear member and the operation of rotating the linear member about the central axis in the extension direction of the linear member, thereby enabling smooth performance of work, such as surgery, using the linear member.

In this case, since the first grip members that grip the linear member are rotatably supported by the second grip members, the linear member can be rotated only by rotating the first grip members without having to rotate the entire device. This allows the device to be reduced in size, and the linear member can be rotated without restrictions on the direction of rotation or the angle of rotation.

It should be understood that the effects of the present disclosure are not limited to the effects described above, but include all effects that can be inferred from the composition of the disclosure described in the detailed description or claims of the present disclosure.

### BRIEF DESCRIPTION OF THE DRAWING

The above and other aspects, features and other advantages of the present disclosure will be more clearly understood from the following detailed description taken in conjunction with the accompanying drawings, in which:
FIG. 1 is a drawing illustrating a linear member moving device according to one embodiment of the present disclosure;
FIG. 2 is a drawing of the linear member moving device according to one embodiment of the present disclosure, arbitrarily separated;
FIG. 3 is an exploded perspective view of a part of the linear member moving device illustrated in FIG. 2;
FIG. 4 is a drawing for explaining disposition and one operation of a pair of first grip members and a pair of second grip members according to one embodiment of the present disclosure;
FIG. 5 is a drawing for explaining the disposition and another operation of the pair of first grip members and the pair of second grip members according to one embodiment of the present disclosure;
FIG. 6 is a drawing for explaining the disposition and still another operation of the pair of first grip members and the pair of second grip members according to one embodiment of the present disclosure;
FIG. 7 is a drawing illustrating one surface of a different form from one surface of the first grip member illustrated in FIGS. 4 to 6;
FIG. 8 is a drawing illustrating an example of a first driver according to one embodiment of the present disclosure;
FIG. 9 is a drawing illustrating another example of the first driver according to one embodiment of the present disclosure;
FIG. 10 is a drawing illustrating still another example of the first driver according to one embodiment of the present disclosure;
FIGS. 11 and 12 are drawings for explaining a push unit according to one embodiment of the present disclosure;
FIGS. 13 to 16 are drawings for explaining a mechanism by which the linear member moving device according to one embodiment of the present disclosure moves a linear member in an extension direction of the linear member;
FIGS. 17 to 18 are drawings for explaining a mechanism by which the linear member moving device according to one embodiment of the present disclosure rotates a linear member about a central axis in the extension direction of the linear member;
FIG. 19 is a drawing for explaining a configuration that may be added to the linear member moving device according to one embodiment of the present disclosure;
FIG. 20 is a perspective view of a first driving module according to another embodiment of the present disclosure viewed from one direction;
FIG. 21 is a drawing of the first driving module according to another embodiment of the present disclosure viewed from another direction;
FIG. 22 is a drawing for explaining the operation of a pair of link units constituting the first driving module according to another embodiment of the present disclosure; and
FIG. 23 is a drawing illustrating a linear member moving system according to one embodiment of the present disclosure.

### DETAILED DESCRIPTION

Hereinafter, with reference to the attached drawings, embodiments of the present disclosure will be described in detail so that those skilled in the art may easily practice the present disclosure. The present disclosure may be implemented in various different forms and is not limited to the embodiments described herein. In order to clearly describe the present disclosure, parts that are not related to the description are omitted in the drawings, and the same reference numerals are assigned to the same or similar components throughout the specification.

The words and terms used in the present specification and claims should not be construed as limited to their usual or dictionary meanings, but should be interpreted as having meanings and concepts consistent with the technical idea of the present disclosure, in accordance with the principles by which the inventor may define terms and concepts in order to best describe his or her disclosure.

Therefore, the embodiments described in the present specification and the configurations illustrated in the drawings correspond to preferred embodiments of the present disclosure and do not represent all of the technical ideas of the present disclosure, and therefore, the configurations may have various equivalents and modified examples that may replace them at the time of filing of the present disclosure.

In the present specification, it should be understood that terms such as "include" or "have" are intended to describe the presence of a feature, number, step, operation, component, part or combination thereof described in the specification, but do not exclude in advance the possibility of the presence or addition of one or more other features, numbers, steps, operations, components, parts or combinations thereof.

When a component is said to be "in front of", "behind", "above", or "below" another component, unless there are special circumstances, it includes not only being positioned "in front of", "behind", "above", or "below" the other component in direct contact with it, but also cases where there are other components intervening therebetween. Furthermore, when a component is said to be "connected" to another component, unless there are special circumstances, it includes cases where they are indirectly connected to each other as well as cases where they are directly connected to each other.

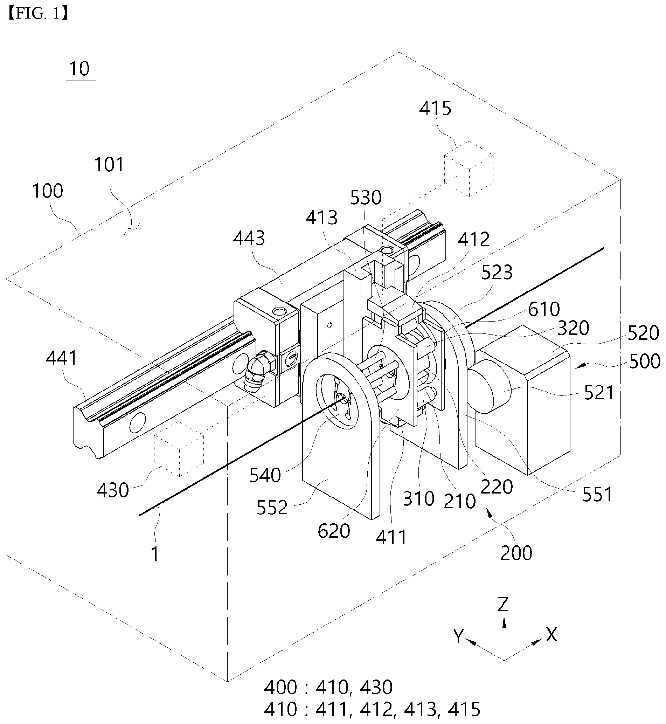

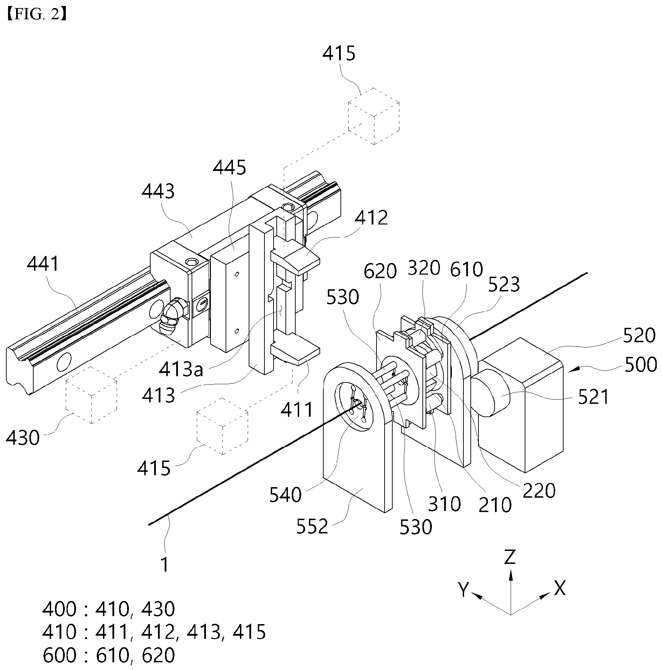

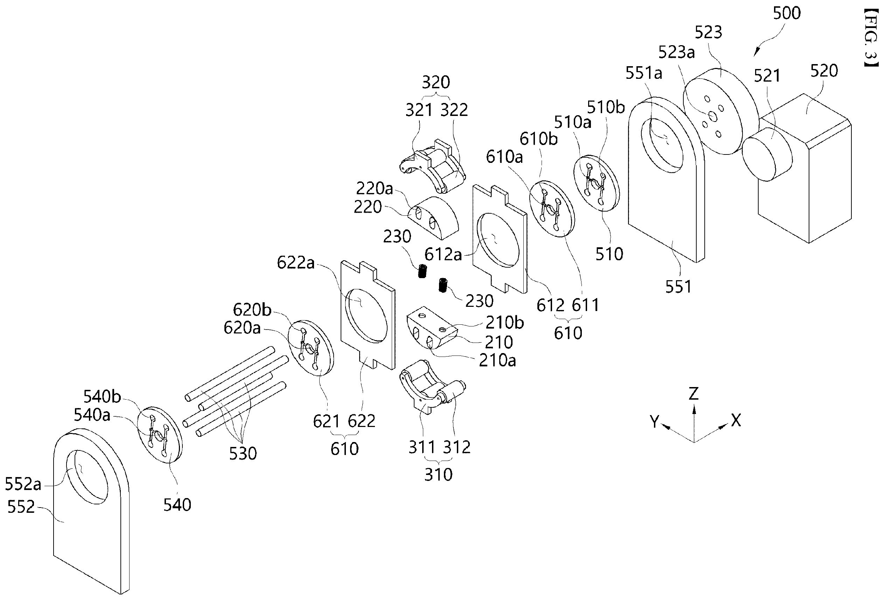

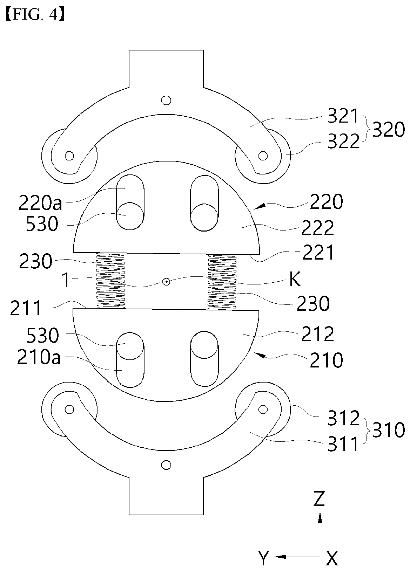

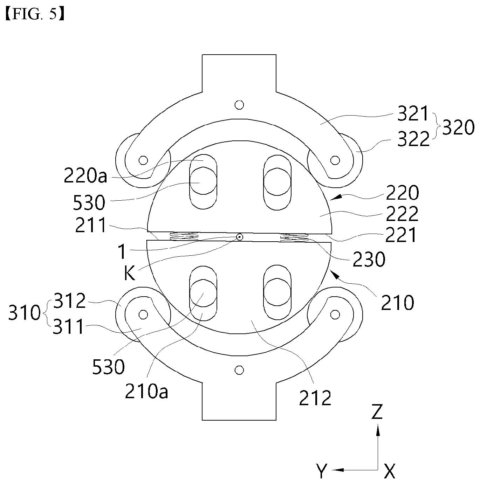

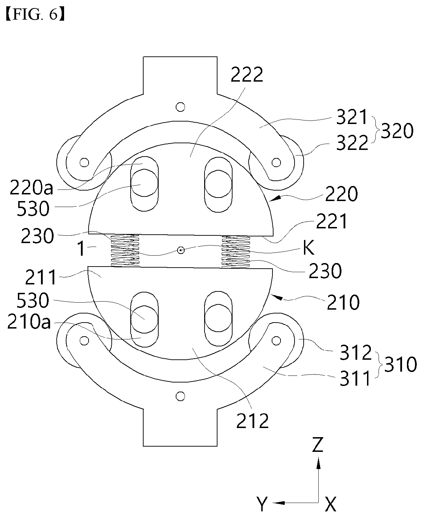

FIG. 1 is a drawing illustrating a linear member moving device according to one embodiment of the present disclosure, and FIG. 2 is a drawing of the linear member moving device according to one embodiment of the present disclosure, arbitrarily separated. FIG. 3 is an exploded perspective view of a part of the linear member moving device illustrated in FIG. 2, FIG. 4 is a drawing for explaining disposition and one operation of a pair of first grip members and a pair of second grip members according to one embodiment of the present disclosure, FIG. 5 is a drawing for explaining the disposition and another operation of the pair of first grip members and the pair of second grip members according to one embodiment of the present disclosure, and FIG. 6 is a drawing for explaining the disposition and still another operation of the pair of first grip members and the pair of second grip members according to one embodiment of the present disclosure.

Referring to FIGS. 1 to 6, a linear member moving device 10 according to one embodiment of the present disclosure is a device that moves a linear member 1 in the extension direction of the linear member 1.

The linear member 1 includes a surgical tool in the form of a tube, such as a catheter, a guide wire, or the like. The linear member 1 has a predetermined length, and FIG. 1 illustrates a portion of the entire linear member 1 that is moved by the linear member moving device 10.

In one embodiment of the present disclosure, the linear member 1 moved by the linear member moving device 10 may have a portion extending in an X-axis direction as illustrated in FIG. 1. For example, linear member penetration holes (such as 510a and 540a in FIG. 3) formed in the linear member moving device 10 for the linear member 1 to move through may be disposed in a row in the X-axis direction.

However, the remaining areas not illustrated in FIG. 1 do not necessarily extend in the X-axis direction and may extend in various shapes, such as a curved shape or a shape with a mixture of curves and straight lines.

The linear member 1 may be moved in the X-axis direction, which is the extension direction of the linear member 1, by the linear member moving device 10.

In this case, the linear member 1 may be moved in a +X direction or a -X direction by the linear member moving device 10 according to one embodiment of the present disclosure.

When a tip of the linear member 1 is in the unillustrated area on the right side of the linear member 1 as seen in FIG. 1, the linear member 1 may be said to move forward when the linear member 1 moves in the +X direction, and the linear member 1 may be said to move backward when the linear member 1 moves in the -X direction.

In one embodiment of the present disclosure, the linear member moving device 10 may move the linear member 1 forward or backward in the extension direction of the linear member 1 as well as rotate the linear member 1 about a central axis K in the extension direction of the linear member 1.

The linear member moving device 10 may include a main body 100, a pair of first grip members 210 and 220, a pair of second grip members 310 and 320, a first driving module 400, and a second driving module 500.

The main body 100 is a structure that directly or indirectly supports the components described below.

The main body 100 is illustrated as having a rectangular parallelepiped shape with an internal space 101 in FIG. 1, but this is merely an example and it is obvious that the main body may have various shapes that directly or indirectly support the configurations described below.

A linear member support portion (not illustrated) that supports a portion of the linear member 1 so that the linear member 1 is extended and disposed in a straight line as illustrated in FIG. 1 may be provided in the main body 100.

In one embodiment of the present disclosure, the pair of first grip members 210 and 220 is disposed to face each other with the linear member 1 therebetween. The pair of grip members operates to grip or release the grip of the linear member 1.

More specifically, the pair of first grip members 210 and 220 moves in a direction toward each other as illustrated in FIG. 5 and grips the linear member 1 by pressing the side surface of the linear member 1. Then, the pair of first grip members 210 and 220 moves in a direction away from each other as illustrated in FIG. 4 and FIG. 6 to release the grip of the linear member 1.

In one embodiment of the present disclosure, the pair of first grip members 210 and 220 may be operatively supported by the main body 100 to grip or release the grip of the linear member 1.

For example, the pair of first grip members 210 and 220 may be supported by a connecting member 530 that extends in the extension direction of the linear member 1 and is supported by the main body 100. Each of the first grip members 210 and 220 may be supported by at least one connecting member 530 to the main body 100.

Connecting member penetration holes 210a and 220a through which the connecting member 530 passes may be formed in the pair of first grip members 210 and 220.

Each of the connecting member penetration holes 210a and 220a may have a slit shape that extends in a direction in which the pair of first grip members 210 and 220 approaches or moves away from each other. In other words, the connecting member penetration holes 210a and 220a may have a slit shape that extends in the direction of approaching or moving away from the linear member 1. For example, the connecting member penetration holes 210a and 220a may extend in the Z-axis direction as seen in FIGS. 3 to 6.

The pair of first grip members 210 and 220 may be operatively supported by the main body 100 by the connecting member 530 and the connecting member penetration holes 210a and 220a to grip or release the grip of the linear member 1.

The gripping and grip-releasing operations of the pair of first grip members 210 and 220 are performed by the pair of second grip members 310 and 320 described later. This will be described later.

According to one embodiment of the present disclosure, at least one elastic body 230 that provides an elastic force in a direction in which the pair of first grip members 210 and 220 move away may be disposed between the pair of first grip members 210 and 220.

When no external force is applied to the pair of first grip members 210 and 220, the pair of first grip members 210 and 220 may easily release their grips from the linear member 1 by the elastic body 230.

An elastic body insertion hole 210b into which an elastic body 230 is inserted may be formed in the pair of first grip members 210 and 220. For reference, in FIG. 3, the elastic body insertion hole formed in the first grip member 220 located in the +Z direction is not visible as it is covered by the body of the first grip member 220.

In one embodiment of the present disclosure, each of the first grip members 210 and 220 may be formed so that at least a portion of one surface 211 and 221 facing the linear member 1 is formed as a plane.

For example, each of the first grip members 210 and 220 may be formed so that one surface 211 and 221 facing the linear member 1 is formed as a flat surface as illustrated in FIGS. 4 to 6.

In this case, the one surface of the first grip members 210 and 220 facing the linear member 1 may be disposed parallel to each other. In this case, regardless of where the linear member 1 is disposed between the pair of first grip members 210 and 220 facing each other, the degree of pressurization on the linear member 1 may be the same.

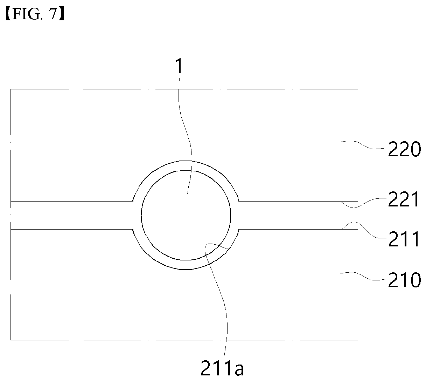

As another example, referring to FIG. 7, insertion grooves 211a and 221a into which the linear member 1 is partially inserted may be formed on one surface 211 and 221 of the first grip members 210 and 220 facing the linear member 1. In this case, one surface 211 and 221 of each of the first grip members 210 and 220 may be formed partially as a plane.

When the linear member 1 is gripped by the pair of first grip members 210 and 220 in a state of being inserted into the insertion grooves 211a and 221a, the linear member 1 is pressed in a form surrounded by the insertion grooves 211a and 221a. In this case, since the contact space between the linear member 1 and the inner surface of the insertion grooves 211a and 221a is large, the pressure is distributed and damage to the linear member 1 may be reduced.

For reference, FIG. 7 is a drawing illustrating one surface different from one surface of the first grip member illustrated in FIGS.4 to 6.

Meanwhile, friction pad insertion grooves into which friction pads are inserted may be formed on one surface of the first grip members facing the linear members, although not illustrated. The linear members may be gripped by the friction pads inserted into the friction pad insertion grooves.

In one embodiment of the present disclosure, each of the first grip members 210 and 220 may be formed so that at least a portion of the other surface 212 and 222 located on the opposite side of the one surface 211 and 221 facing the linear member 1 is formed as a curved surface.

For example, the other surface 212 and 222 located on the opposite side of the one surface 211 and 221 facing the linear member 1 of each of first grip members 210 and 220 may be formed to have an overall curved surface as illustrated in FIGS. 4 to 6. In this case, the pair of first grip members 210 and 220 may rotate smoothly even in a state of being pressurized by the second grip members 310 and 320 described later.

In one embodiment of the present disclosure, each of the first grip members 210 and 220 may have a semicircular shape in a cross section perpendicular to the extension direction of the linear member 1, as illustrated in FIGS. 4 to 6.

In this case, when the pair of first grip members 210 and 220 grips the linear member 1 as illustrated in FIG. 5, the pair of first grip members 210 and 220 may have a cross-section that is generally circular and perpendicular to the extension direction of the linear member 1.

In this case, the pair of second grip members 310 and 320 described later may easily and effectively support the pair of first grip members 210 and 220 in a rotatable manner.

In one embodiment of the present disclosure, the pair of first grip members 210 and 220 may move in the extension direction of the linear member 1 in a state of gripping the linear member 1 as illustrated in FIG. 5. In this case, the linear member 1 may move in the extension direction of the linear member 1 together with the pair of first grip members 210 and 220. This will be described in detail later.

In one embodiment of the present disclosure, the pair of first grip members 210 and 220 may rotate about the central axis K in the extension direction of the linear member 1 in a state of gripping the linear member 1 as illustrated in FIG. 5. In this case, the linear member 1 may rotate about the central axis K in the extension direction. This will be described in detail later.

In one embodiment of the present disclosure, the pair of second grip members 310 and 320 is disposed to face each other with the linear member 1 and the pair of first grip members 210 and 220 interposed therebetween. For example, as seen in FIG. 5, the pair of second grip members 310 and 320 may be disposed spaced apart from each other vertically with the linear member 1 and the pair of first grip members 210 and 220 interposed therebetween.

In one embodiment of the present disclosure, the pair of second grip members 310 and 320 moves in a direction toward each other as illustrated in FIG. 5 to pressurize the pair of first grip members 210 and 220. In other words, the pair of second grip members 310 and 320 moves in a direction toward the linear member 1 to pressurize the pair of first grip members 210 and 220. In this case, the pair of first grip members 210 and 220 may grip the linear member 1 by moving in the direction toward each other.

In one embodiment of the present disclosure, the fact that the pair of second grip members 310 and 320 pressurizes the pair of first grip members 210 and 220 means that the pair of second grip members 310 and 320 pressurizes the pair of first grip members 210 and 220 with a predetermined pressure so that the pair of first grip members 210 and 220 grips the linear member 1.

In one embodiment of the present disclosure, the pair of second grip members 310 and 320 moves away from each other as illustrated in FIG. 4 to depressurize the pair of first grip members 210 and 220. In this case, the pair of first grip members 210 and 220 may move away from each other to release the grip on the linear member 1.

In one embodiment of the present disclosure, the fact that the pair of second grip members 310 and 320 depressurizes the pair of first grip members 210 and 220 means that the pair of second grip members 310 and 320 pressurizes the pair of first grip members 210 and 220 below the predetermined pressure so that the pair of first grip members 210 and 220 releases the grip of the linear member 1.

Hereinafter, for convenience of explanation, a state in which the pair of second grip members 310 and 320 is completely separated from the pair of first grip members 210 and 220 as illustrated in FIG. 4 and the pressing force is 0 is referred to as a completely depressurized state, and a state in which the pair of second grip members 310 and 320 pressurizes the pair of first grip members 210 and 220 with a pressure exceeding 0 and less than a predetermined pressure as illustrated in FIG. 6 is referred to as an incompletely depressurized state.

In one embodiment of the present disclosure, the pair of second grip members 310 and 320 may rotatably support the pair of first grip members 210 and 220 gripping the linear member 1 in a state of pressurizing the first grip members 210 and 220.

Referring to FIGS. 3 to 6, the second grip members 310 and 320 may include second grip member bodies 311 and 321 and rolling members 312 and 322.

The second grip member bodies 311 and 321 are disposed spaced apart from the linear member 1 in the direction perpendicular to the extension direction of the linear member 1.

The second grip member bodies 311 and 321 may be manufactured in a form in which a plurality of plates are combined, as illustrated in FIG. 3. Alternatively, although not illustrated, the second grip member bodies 311 and 321 may be manufactured as a single body.

The side surface of the second grip member bodies 311 and 321 facing the linear member 1 may be formed concavely to wrap around the linear member 1 or the first grip members 210 and 220.

The rolling members 312 and 322 is connected to the second grip member bodies 311 and 321 in a rollable manner.

For example, as illustrated in FIG. 5, when the pair of first grip members 210 and 220 pressurized by the pair of second grip members 310 and 320 rotates in a state of gripping the linear member 1, the rolling members 312 and 322 may roll by contacting the outer peripheral surfaces of the pair of first grip members 210 and 220.

For example, when the pair of first grip members 210 and 220 that has been incompletely depressurized by the pair of second grip members 310 and 320 as illustrated in FIG. 6 rotates in a state of releasing the grip on the linear member 1, the rolling members 312 and 322 may roll by contacting the outer peripheral surfaces of the pair of first grip members 210 and 220.

For example, the rolling members 312 and 322 may be a roller as illustrated in FIG. 3. Alternatively, although not illustrated, the rolling members 312 and 322 may be a ball.

In the rolling members 312 and 322, at least one of the rolling members 312 and 322 may be coupled with each of the second grip member bodies 311 and 321.

In one embodiment of the present disclosure, the pair of second grip members 310 and 320 may be operatively supported by the main body 100 to pressurize or depressurize the pair of first grip members 210 and 220.

For example, the pair of second grip members 310 and 320 may be operably coupled to the first driving module 400 supported by the main body 100. In other words, the pair of second grip members 310 and 320 may be operably supported by the main body 100 via the first driving module 400.

In one embodiment of the present disclosure, the first driving module 400 operates the pair of second grip members 310 and 320 to pressurize or depressurize the pair of first grip members 210 and 220 and operates the pair of second grip members 310 and 320 to move in the extension direction of the linear member 1.

In one embodiment of the present disclosure, the first driving module 400 may include a gripper 410. The gripper 410 provides a driving force for the pair of second grip members 310 and 320 to pressurize or depressurize the pair of first grip members 210 and 220.

The gripper 410 may include a pair of pressing members 411 and 412, a gripper body 413, and a gripper actuator 415.

The pair of pressing members 411 and 412 is connected to the pair of second grip members 310 and 320. The pair of pressing members 411 and 412 may move toward or away from each other in a state of being coupled to the pair of second grip members 310 and 320.

The pair of pressing members 411 and 412 operating in this manner actuates the pair of second grip members 310 and 320 to pressurize or depressurize the pair of first grip members 210 and 220.

The pair of pressing members 411 and 412 may be supported by the gripper body 413 so as to be movable toward or away from each other.

The gripper body 413 may be extended in the direction in which the pair of pressing members 411 and 412 comes closer to or move away from each other. In other words, the gripper body 413 may be formed to extend in the direction perpendicular to the extension direction of the linear member 1.

In this case, a guide groove 413a may be formed in the gripper body 413 to guide the movement of the pair of pressing members 411 and 412.

The gripper actuator 415 provides a driving force for the pair of second grip members 310 and 320 to pressurize or depressurize the pair of first grip members 210 and 220 to the pair of pressing members 411 and 412.

Such a gripper actuator 415 may be operably connected to the pair of pressing members 411 and 412.

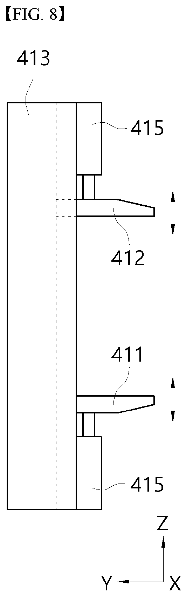

For example, the gripper actuator 415 may be a hydraulic or pneumatic cylinder operably connected to the pressing members 411 and 412 as illustrated in FIG. 8. In this case, two gripper actuators 415 may be used corresponding to the number of pressing members 411 and 412.

Each gripper actuator 415 is supported on one side and the other side of the gripper body 413 and disposed to extend or contract in a direction in which the pair of pressing members 411 and 412 approaches or moves away from each other. An end portion of each gripper actuator 415 may be fixed to each of the pressing members 411 and 412.

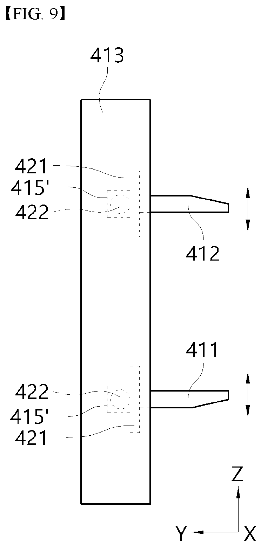

As another example, a gripper actuator 415' may be a motor as illustrated in FIG. 9. In this case, the driving force of the gripper actuator 415', which is a motor, may be transmitted to the pressing members 411 and 412 through a rack 421 and pinion 422.

In this case, the rack 421 is coupled to the pressing members 411 and 412, extends in the movement directions of the pressing members 411 and 412, and is slidably supported by the gripper body 413. The pinion 422 that engages with the rack 421 and the gripper actuator 415' that rotates the pinion 422 are supported by the gripper body 413.

In this case, two gripper actuators 415' may be used corresponding to the number of pressing members 411 and 412.

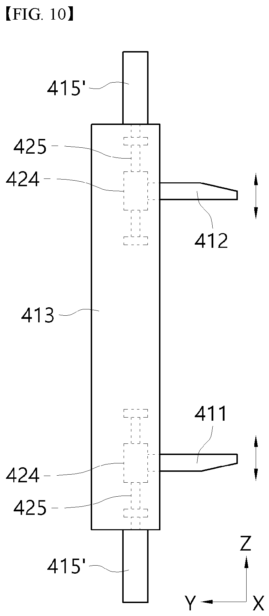

As another example, the gripper actuator 415 which is a motor may be operably connected to the pressing members 411 and 412 by a nut 424 and a screw 425 as illustrated in FIG. 10.

In this case, the nut 424 is coupled to the pressing members 411 and 412 and is slidably supported on the gripper body 413. The screw 425 that engages with the nut 424 is rotatably supported by the gripper body 413. The gripper actuator 415' operatively connected to the screw 425 is supported by the gripper body 413 and rotates the screw 425.

In this case, two gripper actuators 415 which are the motor may be used corresponding to the number of pressing members 411 and 412.

Meanwhile, in FIGS. 8 and 9, two gripper actuators 415' are used to operate the pair of pressing members 411 and 412. However, although not illustrated, in another embodiment, a power transmission member such as a gear, belt, link, or the like may be used to operate the pair of pressing members with one gripper actuator (not illustrated).

In one embodiment of the present disclosure, the first driving module 400 may include an actuator 430 that provides a driving force to move the gripper 410 in the extension direction of the linear member 1.

The actuator 430 may be operably connected to the gripper 410 to move the gripper 410 in the extension direction of the linear member 1.

In one embodiment of the present disclosure, the gripper 410 may be supported by a moving member 443 that is movably coupled to a guide rail 441 extending in the extension direction of the linear member 1. In this case, the guide rail 441 is supported by the main body 100. Alternatively, the guide rail 441 may form a part of the main body.

The moving member 443 may move along the guide rail 441 by the driving force provided by the actuator 430.

For example, the actuator 430 may be a hydraulic or pneumatic cylinder, although not specifically illustrated. In this case, the actuator 430 may be supported by the main body 100 or the guide rail 441.

As another example, the actuator 430 may be a motor, although not specifically illustrated. In this case, the actuator 430 may move the moving member 443 using a rack and pinion or a nut and screw.

In one embodiment of the present disclosure, an intermediate support member 445 may be interposed between the gripper 410 and the moving member 443, but is not limited thereto.

In one embodiment of the present disclosure, the second driving module 500 is supported by the main body 100 so as to rotate the pair of first grip members 210 and 220 about the central axis K in the extension direction of the linear member 1.

The second driving module 500 may include a rotating member 510, a motor 520, and a connecting member 530.

The rotating member 510 is disposed spaced apart from the pair of first grip members 210 and 220 in the extension direction of the linear member 1. The rotating member 510 is supported by the main body 100 so as to rotate about the central axis K in the extension direction of the linear member 1.

The rotating member 510 may have a disk shape.

The rotating member 510 may be rotatably supported by a bracket 551. The bracket 551 may form a part of the main body 100 or may be manufactured separately and coupled to the main body 100. A rotating member coupling hole 551a to which the rotating member 510 is rotatably coupled may be formed in the bracket 551.

The rotating member 510 may be rotatably coupled to the rotating member coupling hole 551a via a bearing (not illustrated).

A linear member penetration hole 510a through which the linear member 1 can move is formed in the rotating member 510.

The motor 520 provides a rotational driving force to rotate the rotating member 510. The motor 520 is operably connected to the rotating member 510.

For example, the motor 520 may be operatively connected to the rotating member 510 through a driving gear 521 coupled to the motor 520 as illustrated in FIGS. 2 and 3, and a driven gear 523 coupled to the rotating member 510 and meshed with the driving gear 521. In this case, a linear member penetration hole 523a through which the linear member 1 passes may be formed in the driven gear 523.

In another example, although not illustrated, the motor 520 may be operably connected to the rotating member 510 via a belt or pulley.

The connecting member 530 interconnects the rotating member 510 and the pair of first grip members 210 and 220.

The connecting member 530 may have a bar shape extending in the extension direction of the linear member 1.

In one embodiment of the present disclosure, at least one connecting member 530 is provided for each of the first grip members 210 and 220.

For example, two connecting members 530 may be provided for each of the first grip members 210 and 220 as illustrated in FIGS. 3 to 6. The two connecting members 530 connected to each of the first grip members 210 and 220 may be connected to the first grip members 210 and 220 so as to be symmetrical about the central axis K in the extension direction of the linear member 1.

In this case, when the two connecting members 530 rotate about the central axis K in the extension direction of the linear member 1, the first grip members 210 and 220 connected to the two connecting members 530 may rotate stably.

In one embodiment of the present disclosure, the connecting member 530 penetrates the first grip members 210 and 220 and is connected to the first grip members 210 and 220. In this case, the connecting member 530 may not only rotate the first grip members 210 and 220, but also guide the first grip members 210 and 220 in the extension direction of the linear member 1.

The connecting member penetration holes 210a and 220a are formed in the first grip members 210 and 220. The connecting member penetration holes 210a and 220a may extend in the direction in which the pair of first grip members 210 and 220 come closer to or farther away from each other.

In this case, the connecting member 530 may perform the role of guiding the pair of first grip members 210 and 220 during the gripping operation or gripping-release operation of the pair of first grip members 210 and 220.

One end portion of the connecting member 530 is fixedly supported on the rotating member 510. In this case, a connecting member coupling hole 510b to which one end portion of the connecting member 530 is coupling may be formed in the rotating member 510.

Moreover, the other end portion of the connecting member 530 may be fixedly supported on an auxiliary rotating member 540. In this case, a connecting member coupling hole 540b to which the other end portion of the connecting member 530 is coupled may be formed in the auxiliary rotating member 540.

The auxiliary rotary member 540 is disposed spaced apart from the rotating member 510 in the extension direction of the linear member 1.

In this regard, the connecting member 530 may support various movements of the first grip members 210 and 220, such as movement in the extension direction of the linear member 1, movement for gripping the linear member 1, and rotational movement about the central axis in the extension direction of the linear member 1, even in a state where one end portion of the connecting member 530 is fixedly supported by the rotating member 510.

In this situation, when the other end portion of the connecting member 530 is fixedly supported by the auxiliary rotating member 540, various movements of the first grip members 210 and 220 may be stably supported.

The auxiliary rotating member 540 may have a disk shape.

The auxiliary rotating member 540 may be rotatably supported by a bracket 552. The bracket 552 may form a part of the main body 100 or may be manufactured separately and coupled to the main body 100. A rotating member coupling hole 552a to which the auxiliary rotating member 540 is rotatably coupled may be formed in the bracket 552.

The auxiliary rotating member 540 may be rotatably coupled to the rotating member coupling hole 552a via a bearing (not illustrated).

The linear member penetration hole 540a through which the linear member 1 can move is formed in the auxiliary rotary member 540.

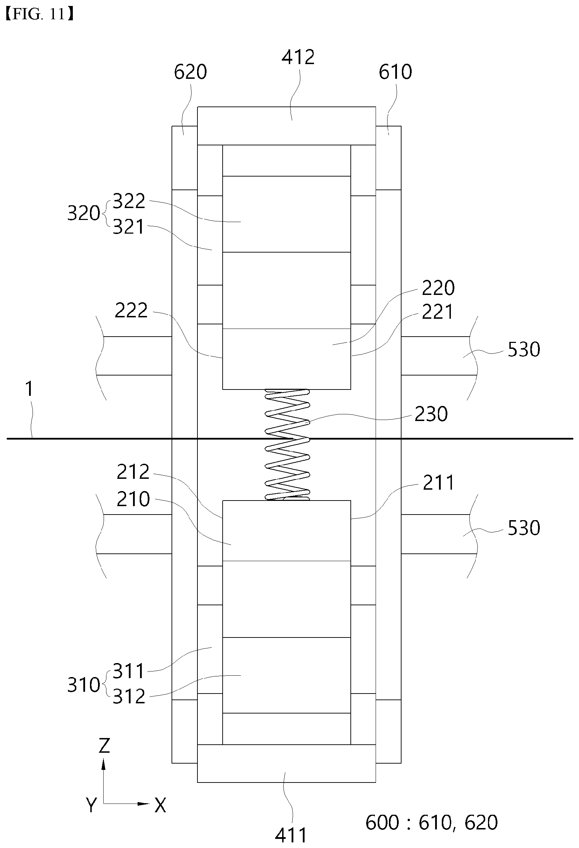

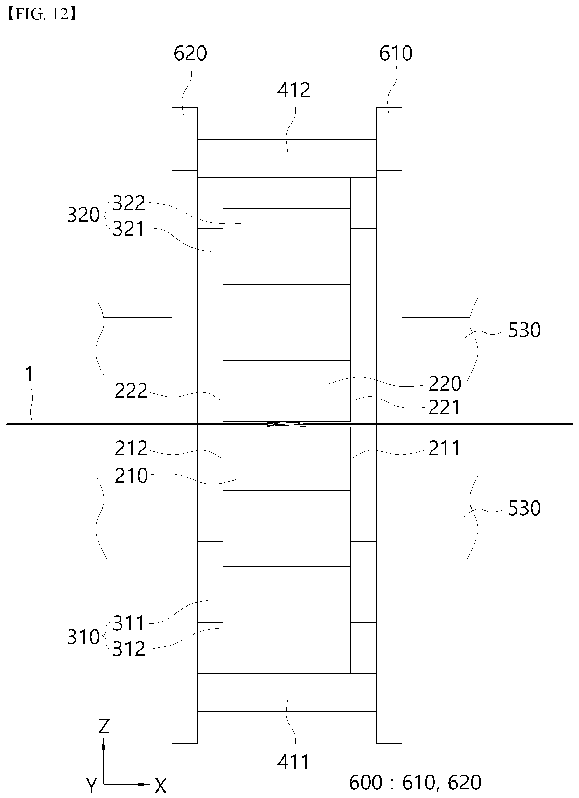

FIGS. 11 and 12 are drawings for explaining a push unit according to one embodiment of the present disclosure. Referring to FIGS. 2, 3, 11 and 12, the linear member moving device 10 according to one embodiment of the present disclosure may further include a push unit 600.

The push unit 600 is operatively supported by the main body 100 so as to push the pair of first grip members 210 and 220 in the movement direction of the pair of second grip members 310 and 320 when the pair of second grip members 310 and 320 moves in the extension direction of the linear member 1 in a completely depressurized state with respect to the pair of first grip members 210 and 220 as illustrated in FIG. 4 and FIG. 11.

In this regard, when the pair of second grip members 310 and 320 moves in the extension direction of the linear member 1 in a state of pressurizing the pair of first grip members 210 and 220 as illustrated in FIG. 5 and FIG. 12, the pair of first grip members 210 and 220 may move together with the pair of second grip members 310 and 320.

Moreover, when the pair of second grip members 310 and 320 moves in the extension direction of the linear member 1 in the incompletely depressurized state with respect to the pair of first grip members 210 and 220 as illustrated in FIG. 6, the pair of first grip members 210 and 220 may move together with the pair of second grip members 310 and 320 by the pressing force provided by the pair of second grip members 310 and 320.

However, when the pair of second grip members 310 and 320 move in the extension direction of the linear member 1 in the completely depressurized state with respect to the pair of first grip members 210 and 220 as illustrated in FIG. 4 and FIG. 11, the pair of first grip members 210 and 220 may not move together with the pair of second grip members 310 and 320 because there is no pressing force provided by the pair of second grip members 310 and 320. In this case, the push unit 600 pushes the pair of first grip members 210 and 220 in the movement direction with the pair of second grip members 310 and 320 to move them together.

In one embodiment of the present disclosure, the push unit 600 may include a pair of push members 610 and 620 spaced apart from each other in the extension direction of the linear member 1 with the pair of first grip members 210 and 220 interposed therebetween.

The pair of push members 610 and 620 may be disposed to face both sides 201 and 202 of the pair of first grip members 210 and 220 in the extension direction of the linear member 1.

The pair of push members 610 and 620 may be supported by being interconnected by a separate connecting support member, although this is not illustrated.

In one embodiment of the present disclosure, each of the push members 610 and 620 has a plate shape as illustrated in FIGS. 2, 3 and 11, and is depicted as having a shape that covers not only both sides 201 and 202 of the pair of first grip members 210 and 220 in the extension direction of the linear member 1, but also both sides of the pair of second grip members 310 and 320 in the extension direction of the linear member 1.

However, each of the push members 610 and 620 is not limited to the shape as described above, and may have various shapes that may push and move the pair of first grip members 210 and 220 in a state where the pair of second grip members 310 and 320 completely releases pressure with respect to the pair of first grip members 210 and 220.

In one embodiment of the present disclosure, the push unit 600 may move in the extension direction of the linear member 1 by the driving force of the first driving module 400 that moves the pair of second grip members 310 and 320 in the extension direction of the linear member 1.

In this case, the push unit 600 may be supported by a portion of the first driving modules 400 that moves in the extension direction of the linear member 1 together with the pair of second grip members 310 and 320. In this case, the push unit 600 may be operatively supported by the main body 100 via the driving module supported by the main body 100.

For example, the pair of push members 610 and 620 constituting the push unit 600 may be fixedly supported on the gripper body 413 of the first driving module 400.

As another example, or although not illustrated, the pair of push members 610 and 620 constituting the push unit 600 may be fixedly supported by the moving member 443 of the first driving module 400.

Alternatively, in the push unit 600, although not illustrated, the pair of push members 610 and 620 may be moved by a separate driving module other than the first driving module 400.

In one embodiment of the present disclosure, the pair of push members 610 and 620 are disposed spaced apart from each other by a predetermined distance in the extension direction of the linear member 1.

In one embodiment of the present disclosure, the pair of first grip members 210 and 220 between the pair of push members 610 and 620 may perform the gripping or grip-releasing operation, and the pair of push members 610 and 620 may guide the operations of the pair of first grip members 210 and 220.

In this case, as illustrated in FIGS. 11 and 12, both sides 201 and 202 of the pair of first grip members 210 and 220 in the extension direction of the linear member 1 and the pair of push members 610 and 620 may be spaced apart by a predetermined interval.

In one embodiment of the present disclosure, the pair of push members 610 and 620 is formed in a shape that covers both sides of the pair of first grip members 210 and 220 in the extension direction of the linear member 1.

Furthermore, the pair of push members 610 and 620 is formed in a form that covers both sides of the pair of second grip members 310 and 320 in the extension direction of the linear member 1.

In this case, the pair of second grip members 310 and 320 between the pair of push members 610 and 620 may perform a pressurizing or depressurizing operation, and the pair of push members 610 and 620 may guide the operation of the pair of second grip members 310 and 320.

For example, in FIGS. 11 and 12, both sides of the pair of second grip members 310 and 320 in the extension direction of the linear member 1 may contact the push members 610 and 620. In this case, the contact surfaces of the pair of second grip members 310 and 320 and the pair of push members 610 and 620 may be processed to have a low coefficient of friction.

As another example, although not illustrated, both sides of the pair of second grip members 310 and 320 in the extension direction of the linear member 1 may be spaced apart from the pair of push members 610 and 620 by a predetermined distance.

In one embodiment of the present disclosure, the push members 610 and 620 may include rotary plates 611 and 621 and push member bodies 612 and 622, respectively.

Linear member penetration holes 610a and 620a through which the linear member 1 passes may be formed in the rotary plates 611 and 621. The rotary plates 611 and 621 may rotate about the linear member penetration holes 610a and 620a.

The rotary plates 611 and 621 may be disposed to face one of both sides 201 and 202 of the pair of first grip members 210 and 220 in the extension direction of the linear member 1. In this case, the rotary plates 611 and 621 may be a part that pushes the pair of first grip members 210 and 220.

The push member bodies 612 and 622 rotatably support the rotary plates 611 and 621. The push member bodies 612 and 622 may be formed with rotary plate coupling holes 612a and 622a to which the rotary plates 611 and 621 is rotatably coupled. The rotary plates 611 and 621 may be rotatably coupled to the rotary plate coupling holes 612a and 622a via a bearing.

Connecting member penetration holes 610b and 620b through which the connecting member 530 constituting the second driving module 500 passes are formed in the rotary plates 611 and 621. In this case, the connecting member 530 positioned penetrating through the connecting member penetration holes 610b and 620b may guide the pair of push members 610 and 620 including the rotary plates 611 and 621 in the extension direction of the linear member 1.

The connecting member penetration holes 610b and 620b may be formed around the linear member penetration holes 610a and 620a.

In another embodiment, although not illustrated, the pair of push members may include a push member body having a shape similar to the push member bodies 612 and 622 of FIG. 3, but without the rotary plates 611 and 621 of FIG. 3. In this case, the push member body may have an integrated through hole (not illustrated) through which both the connecting member 530 and the linear member 1 pass.

In this case, the through hole may be formed so that when the push member body moves in the extension direction of the linear member 1 in a state where the pair of first grip members 210 and 220 release the grip of the linear member 1, the peripheral area of the through hole is caught by the pair of first grip members 210 and 220.

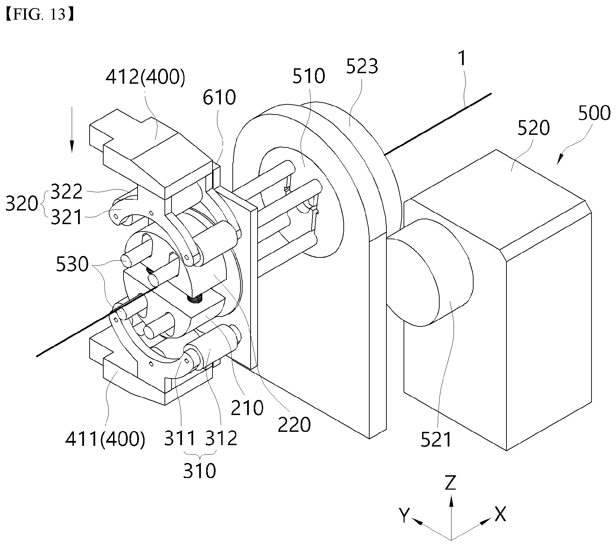

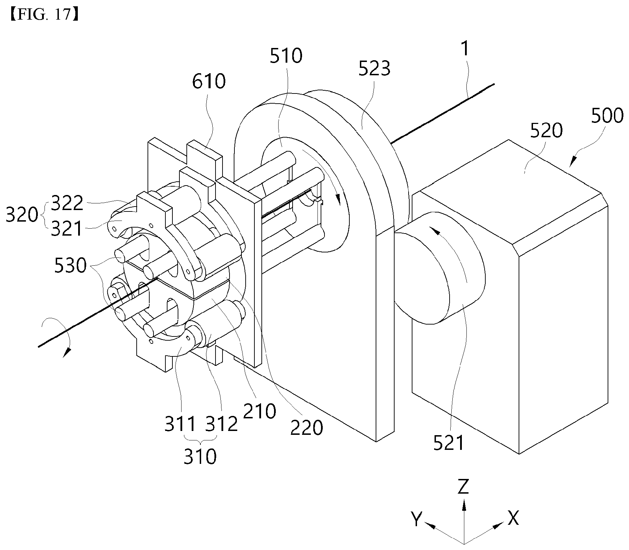

FIGS. 13 to 16 are drawings for explaining a mechanism by which the linear member moving device according to one embodiment of the present disclosure moves the linear member in the extension direction of the linear member, and FIGS. 17 to 18 are drawings for explaining a mechanism by which the linear member moving device according to one embodiment of the present disclosure rotates the linear member about the central axis in the extension direction of the linear member.

For reference, some components of the linear member moving device 10 according to one embodiment of the present disclosure are omitted in FIGS. 13 to 18.

Hereinafter, with reference to FIGS. 13 to 16, an operating mechanism of the linear member moving device 10 according to one embodiment of the present disclosure for moving the linear member 1 in the extension direction of the linear member 1 will be described.

Referring to FIG. 13, the pair of second grip members 310 and 320 is placed in a state where the second grip members completely release the gripping with respect to the pair of first grip members 210 and 220. In this case, the pair of pressing members 411 and 412 of the first driving module 400 operate so that the pair of second grip members 310 and 320 pressurizes the pair of first grip members 210 and 220.

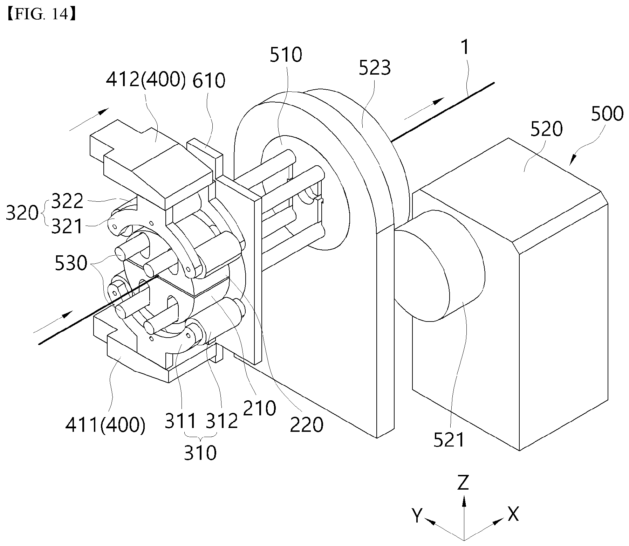

Due to the operation of the pair of pressing members 411 and 412 like this, a pair of first grip members 210 and 220 may grip the linear member 1 as illustrated in FIG. 14.

Referring to FIG. 14, in a state where the pair of first grip members 210 and 220 grips the linear member 1, the pair of pressing members 411 and 412 of the first driving module 400 operates to move the pair of second grip members 310 and 320 in the +X direction, which is one direction of the extension directions of the linear member 1.

In this case, the linear member 1 gripped by the pair of first grip members 210 and 220 may move in the movement direction of the pair of pressing members 411 and 412 or in the extension direction of the linear member 1.

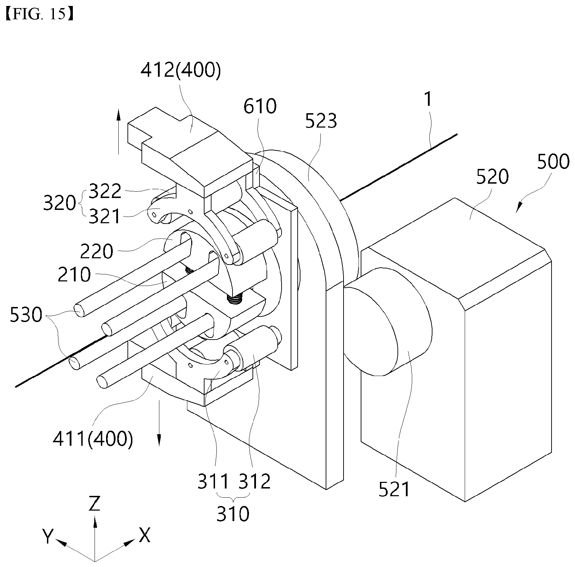

Due to the operation of the pair of pressing members 411 and 412 as described above, the pair of second grip members 310 and 320, the pair of first grip members 210 and 220, and the linear member 1 may move by a predetermined distance in the +X direction and stop as illustrated in FIG. 15.

Referring to FIG. 15, the pair of pressing members 411 and 412 of the pair of first driving modules 400 operates to cause the pair of second grip members 310 and 320 to depressurize the pair of first grip members 210 and 220. For reference, FIG. 15 illustrates a state in which the pair of second grip members 310 and 320 completely depressurizes the pair of first grip members 210 and 220.

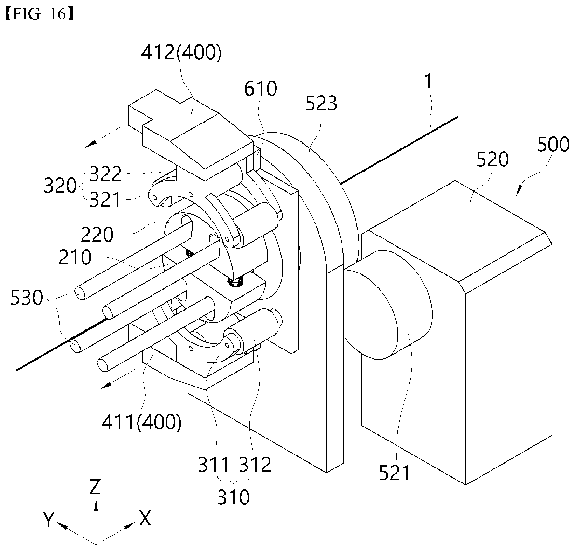

Due to the operation of the pair of pressing members 411 and 412 like this, the pair of first grip members 210 and 220 may release the grip of the linear member 1 as illustrated in FIG. 16.

Referring to FIG. 16, in a state where the pair of first grip members 210 and 220 release the grip of the linear member 1, the pair of pressing members 411 and 412 of the first driving module 400 operates to move the pair of second grip members 310 and 320 in the other direction of the extension directions of the linear member 1, that is, in the -X direction.

In this case, the push members 610 and 620 supported by the gripper body 413 of the first driving module 400 may move the pair of first grip members 210 and 220 completely released from the pair of second grip members 310 and 320 in the -X direction, which is the movement direction of the pair of pressing members 411 and 412.

Meanwhile, FIGS. 13 to 16 illustrate a mechanism by which the linear member moving device 10 according to one embodiment of the present disclosure moves the linear member 1 in one direction of the extension directions of the linear member 1, that is, in the +X direction.

However, it is obvious to those skilled in the art that the linear member moving device 10 according to one embodiment of the present disclosure may move the linear member 1 in the other direction of the extension direction of the linear member 1, that is, in the -X direction, by a mechanism substantially the same as the mechanism described in FIGS. 13 to 16.

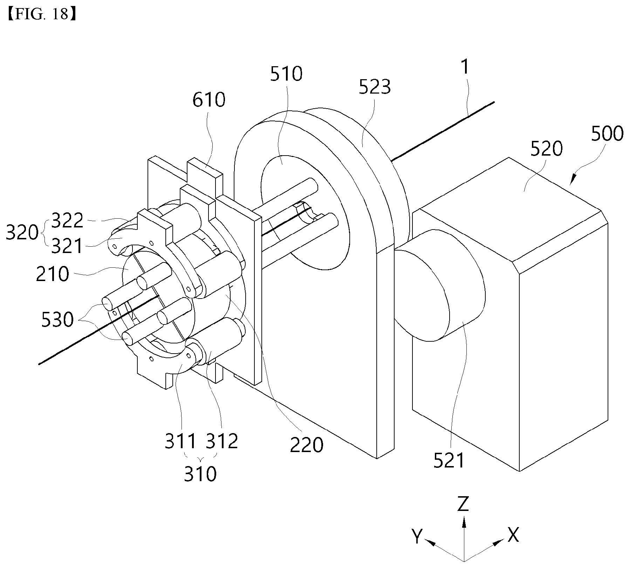

Hereinafter, with reference to FIGS. 17 and 18, the operating mechanism by which the linear member moving device 10 according to one embodiment of the present disclosure rotates the linear member 1 about the central axis in the extension direction of the linear member 1 will be described.

Referring to FIG. 17, due to the operation of the pair of pressing members 411 and 412 of the first driving module 400, the pair of second grip members 310 and 320 may pressurize the pair of first grip members 210 and 220, and in series, the pair of first grip members 210 and 220 may grip the linear member 1.

When the motor 520 of the second driving module 500 operates in a state where the pair of first grip members 210 and 220 grips the linear member 1, the driving force of the motor 520 is transmitted to the pair of first grip members 210 and 220 through the driving gear 521, the driven gear 523, and the connecting members 530.

The pair of first grip members 210 and 220 that receive driving force from the motor 520 may rotate in one direction about the central axis in the extension direction of the linear member 1 as illustrated in FIG. 18. In this process, the linear member 1 gripped by the pair of first grip members 210 and 220 may also rotate together.

The rotation direction of the pair of grip members and the linear member 1 may vary depending on the direction of the rotational driving force generated from the motor 520.

Meanwhile, the linear member moving device 10 according to one embodiment of the present disclosure may separately or simultaneously perform the operation of moving the linear member 1 in the extension direction of the linear member 1 and the operation of rotating the linear member 1 about the central axis in the extension direction of the linear member 1.

The linear member moving device 10 according to one embodiment of the present disclosure as described above performs separately or simultaneously the operation of linearly moving the linear member 1 in the extension direction of the linear member 1 and the operation of rotating the linear member 1 about the central axis of the extension direction of the linear member 1, thereby enabling smooth performance of work, such as surgery, using the linear member 1.

In this case, since the first grip members 210 and 220 that grip the linear member 1 is rotatably supported by the second grip members 310 and 320, the linear member 1 may be rotated by only rotating the first grip members 210 and 220 without having to rotate the entire device. As a result, the size of the device may be reduced, and the linear member 1 may be rotated without restrictions on the direction of rotation or the angle of rotation.

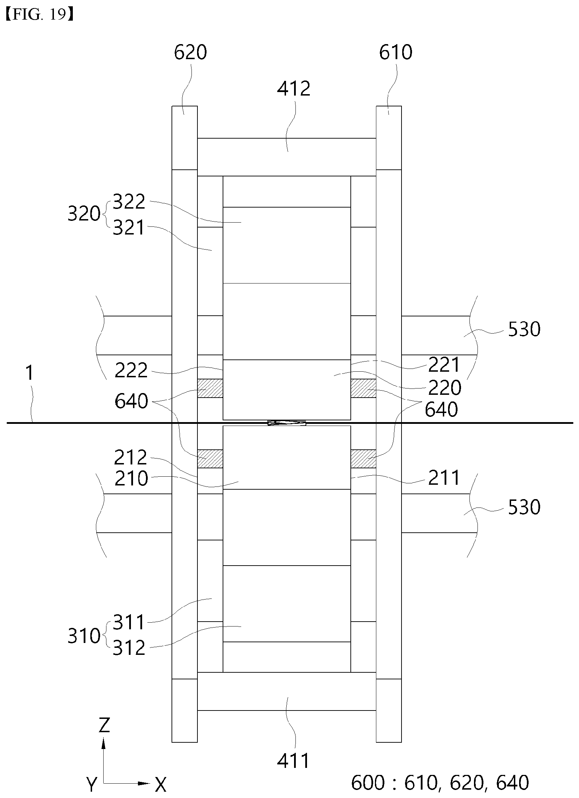

FIG. 19 is a drawing for explaining a configuration that may be added to a linear member moving device according to one embodiment of the present disclosure. For reference,

FIG. 19 illustrates a state in which, similar to FIG. 12, the pair of second grip members pressurizes the pair of first grip members 210 and 220 so that the pair of first grip members 210 and 220 grip the linear member.

Referring to FIG. 19, the linear member moving device 10 may further include a force sensor 640.

The force sensor 640 may be interposed between both sides 201 and 202 of the pair of first grip members 210 and 220 in the extension direction of the linear member 1 and the pair of push members 610 and 620.

The force sensor 640 measures the pressure between the pair of first grip members 210 and 220 and the pair of push members when the pair of first grip members 210 and 220 move in the extension direction of the linear member in a state of gripping the linear member 1.

The pressure measured by the force sensor 640 is proportional to or substantially equal to the tension applied to the linear member 1 when the pair of first grip members 210 and 220 move in the extension direction of the linear member 1 in a state of gripping the linear member. The tension of the linear member 1 may be adjusted based on the measured pressure of the force sensor 640.

Meanwhile, the first driving module 400 according to one embodiment of the present disclosure is configured to include the gripper 410 and the actuator 430 as illustrated in FIGS. 1 and 2.

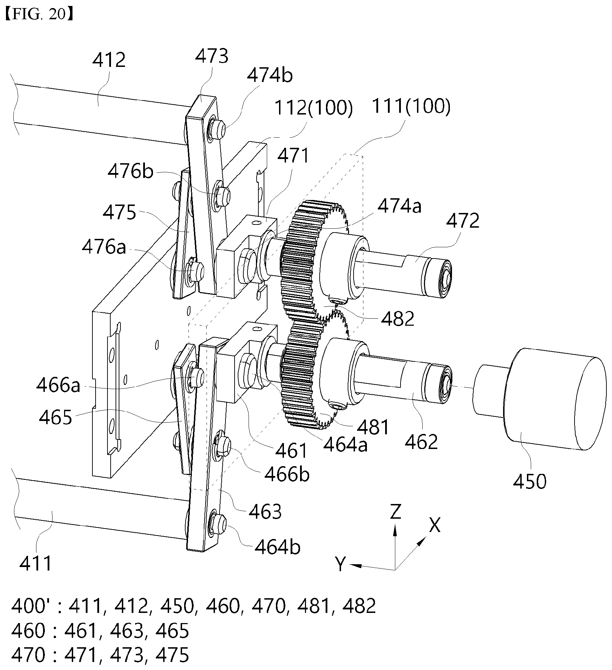

Alternatively, the first driving module 400' according to another embodiment of the present disclosure may include the pair of pressing members 411 and 412, a driving motor 450, and a pair of link units 460 and 470 as illustrated in FIGS. 20 to 22.

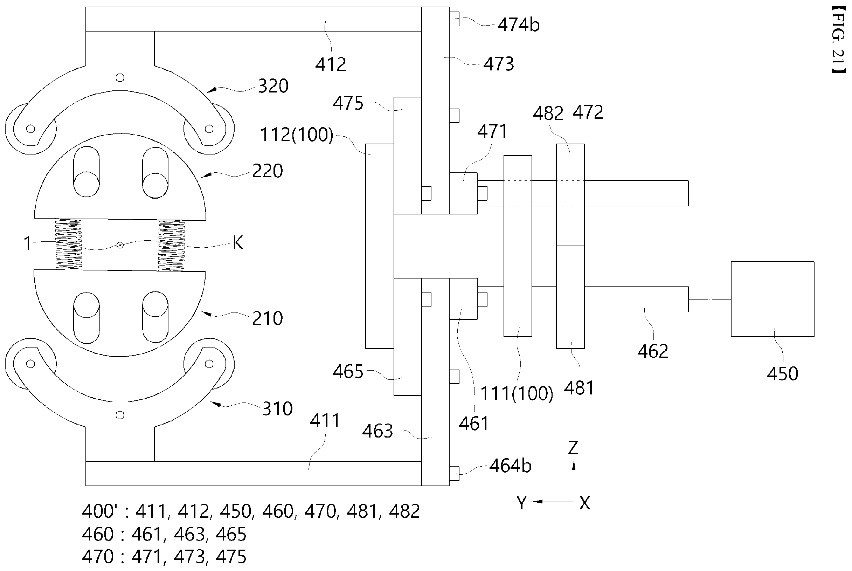

For reference, FIG. 20 is a perspective view of a first driving module according to another embodiment of the present disclosure when viewed from one direction, and FIG. 21 is a drawing of the first driving module according to another embodiment of the present disclosure viewed from another direction. In FIG. 20, a pair of pressing members is omitted.

The pair of pressing members 411 and 412 may be coupled to the pair of second grip members 310 and 320.

The driving motor 450 provides driving force to operate the pair of pressing members 411 and 412.

The pair of link units 460 and 470 operably connects the driving motor 450 and the pressing members 411 and 412. The pair of link units 460 and 470, which receive the driving force of the driving motor 450, operate the pair of pressing members 411 and 412 so that the pair of pressing members 411 and 412 move along a first trajectory in which the pair of pressing members 411 and 412 move in one direction of the extension directions of the linear member 1 and a second trajectory in which the pair of pressing members 411 and 412 move away from and toward the linear member 1 while moving in the other direction of the extension directions of the linear member 1. This will be described later.

For example, the link units 460 and 470 include driving links 461 and 471, connecting links 463 and 473, and auxiliary links 465 and 475.

One end portion of each of the driving links 461 and 471 may be rotated by the driving motor 450. In this case, the driving links 461 and 471 may receive the driving force of the driving motor 450 and rotate about a third direction that is perpendicular to both a first direction, which is the extension direction of the linear member 1, and a second direction, which is the direction in which the pair of second grip members 310 and 320 approach or move away from each other.

In other words, the driving links 461 and 471 may rotate about the lateral direction of the extension direction of the linear member 1 by receiving the driving force of the driving motor 450.

One end portion of each of the driving links 461 and 471 may be rotatably supported by the main body 100.

For example, rotary shafts 462 and 472 coupled to one end portion of each of the driving links 461 and 471 may be rotatably supported by a first support member 111 constituting a part of the main body 100 by a bearing (not illustrated).

In this case, the rotary shafts 462 and 472 are fixedly connected to one end portion of each of the driving links 461 and 471, and may rotate together with the driving links 461 and 471 when the rotary shafts 462 and 472 are rotated by the driving motor 450.

The connecting links 463 and 473 connect the pressing members 411 and 412 and the driving links 461 and 471. In this case, one end portion of each of the connecting links 463 and 473 may be pivotally connected to the other end portion of the driving links 461 and 471, and the other end portion of each of the connecting links 463 and 473 may be pivotally connected to each of the pressing members 411 and 412.

For example, one end portion of each of the connecting links 463 and 473 and the other end portion of each of the driving links 461 and 471 may be pivotally connected by pivot shafts 464a and 474a, and the other end of each of the connecting links 463 and 473 and each of the pressing members 411 and 412 may be connected to pivot shafts 464b and 474b.

The auxiliary links 465 and 475 may be formed so that one end portion of each of the auxiliary links is pivotally connected to the main body 100 and the other end thereof is pivotally connected to the central region of each of the connecting links 463 and 473.

For example, one end portion of each of the auxiliary links 465 and 475 and the second support member 112 constituting a part of the main body 100 may be pivotally connected by pivot shafts 466a and 476a, and the other end portion of each of the auxiliary links 465 and 475 and the central region of each of the connecting links 463 and 473 may be pivotally connected to pivot shafts 466b and 476b.

In the present embodiment, the driving links 461 and 471 of the link units 460 and 470 may rotate in directions opposite to each other by receiving driving force from the driving motor 450.

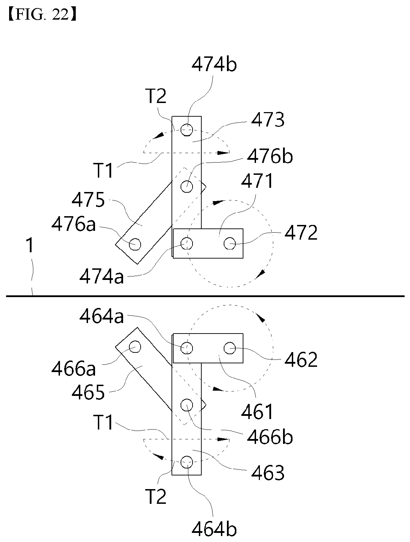

In this case, the pair of link units 460 and 470 that receives the driving force of the driving motor 450 operates in a symmetrical structure with respect to the linear member 1 when viewed from the third direction, and the pair of pressing members 411 and 412 coupled with the pair of link units 460 and 470 may move along the first and second trajectories, but may move in a symmetrical structure with respect to the linear member 1 when viewed from the third direction. This will be described in detail with reference to FIG. 22.

In the present embodiment, the first driving module 400' may further include a pair of link drive gears 481 and 482.

The link drive gears 481 and 482 may be axial-coupled to rotate together with one end portion of each of the driving links 461 and 471 of the link units 460 and 470. In this case, the link drive gears 481 and 482 may be axial-coupled to the rotary shafts 462 and 472 that are axial-coupled with one end portion of each of the driving links 461 and 471.

In this case, when the rotary shafts 462 and 472 rotates, the link drive gears 481 and 482 and the driving links 461 and 471 may rotate together.

The pair of link drive gears 481 and 482 may mesh with each other and rotate in opposite directions. In this case, one of the pairs of link drive gears 481 and 482 is operably connected to the driving motor 450 to provide the driving force.

In this case, it is economical to use one driving motor 450 to rotate the pair of interlocked link drive gears 481 and 482.

The driving motor 450 may transmit driving force to one of the pair of link drive gears 481 and 482 by a power transmission means such as a gear, pulley, belt, or the like. Alternatively, the driving motor 450 may transmit driving force by being directly connected to the rotary shaft that is axial-coupled to one of the pair of link drive gears 481 and 482.

For example, the driving motor 450 may transmit the driving force indirectly to the link drive gear 481 located below in FIGS. 20 and 21 by a power transmission means or directly without the power transmission means.

FIG. 22 is a drawing for explaining the operation of the pair of link units constituting the first driving module according to another embodiment of the present disclosure.

Referring to FIGS. 20 to 22, when the driving force of the driving motor 450 is transmitted to the driving links 461 and 471 of the link units 460 and 470, the driving links 461 and 471 rotate in the directions different from each other.

For example, as illustrated in FIG. 22, the driving link 461 located below may rotate counterclockwise about the rotary shaft 462, and the driving link 471 located above may rotate clockwise about the rotary shaft 472.

In this case, the pressing members 411 and 412 connected to the other end of the connecting links 463 and 473 connected to the driving links 461 and 471 by the pivot shaft 464b and 474b may move along a first trajectory T1 moving in one direction of the extension directions of the linear member 1 and a second trajectory T2 moving away from and then approaching the linear member 1 while moving in the other direction of the extension directions of the linear member 1.

In this case, the first trajectory T1 may have a straight-line shape, and the second trajectory T2 may have a semi-elliptical shape.

The first trajectory T1 and second trajectory T2 operations of the pressing members 411 and 412 have a symmetrical structure about the linear member 1 when viewed from the third direction, as illustrated in FIG. 22.

In the process in which the pair of pressing members 411 and 412 move along the first trajectory T1, the pair of pressing members 411 and 412 pressurizes the pair of second grip members 310 and 320 to a pressure equal to or more than a predetermined pressure.

In this case, the pair of first grip members 210 and 220 may be in a state of gripping the linear member 1, and accordingly, the pair of pressing members 411 and 412, the pair of second grip members 310 and 320, the pair of first grip members 210 and 220, and the linear member 1 may move together in one direction of the extension directions of the linear member 1.

Here, one direction of the extension directions of the linear member 1 may be the right direction as seen in FIG. 22. However, one direction of the extension directions of the linear member 1 may be the left direction as seen in FIG. 22 depending on the rotation direction of the driving motor 450.

In the process of moving along the second trajectory T2 of the pair of pressing members 411 and 412, the pair of pressing members 411 and 412 pressurizes the pair of second grip members 310 and 320 below a predetermined pressure.

In this case, the pair of first grip members 210 and 220 may be in a state of releasing the grip of the linear member 1, and accordingly, the pair of pressing members 411 and 412 and the pair of second grip members 310 and 320 may move together in the other direction of the extension directions of the linear member 1.

Here, the other direction of the extension directions of the linear member 1 may be the left direction as seen in FIG. 22. However, the other direction of the extension directions of the linear member 1 may be the right direction as seen in FIG. 22 depending on the rotation direction of the driving motor 450.

In addition to the first driving module 400 and 400' described above, it goes without saying that various forms of first driving modules may be proposed in which the pair of second grip members 310 and 320 pressurizes or depressurizes the pair of first grip members 210 and 220 and provides driving force for movement in the extension direction of the linear member 1.

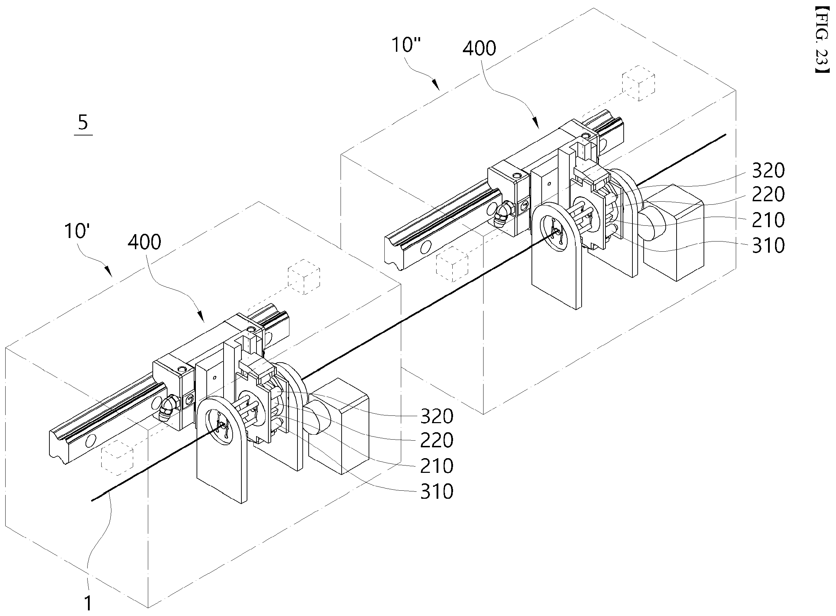

Meanwhile, FIG. 23 is a drawing illustrating a linear member moving system according to one embodiment of the present disclosure.

Referring to FIG. 23, a linear member moving system 5 according to one embodiment of the present disclosure may include a pair of linear member moving devices 10' and 10".

A first linear member moving device 10' and a second linear member moving device 10" of FIG. 23 are identical to the previous linear member moving device 10 and are illustrated with only different identification numbers. Therefore, a specific description of the configuration, structure, operation, modifications, or the like of the first linear member moving device 10' and the second linear member moving device 10" is replaced with the description of the linear member moving device 10 described above.

The linear member moving system 5 according to one embodiment of the present disclosure is operated so that when the first driving module 400 of the first linear member moving device 10' provides the driving force for moving in one direction of extension directions of the linear member 1 in a state where the pair of second grip members 310 and 320 of the first linear member moving device 10' pressurizes the pair of first grip members 210 and 220 of the first linear member moving device 10', the first driving module 400 of the second linear member moving device 10" provides the driving force for moving in the other direction of the extension directions of the linear member 1 in a state where the pair of second grip members 310 and 320 of the second linear member moving device 10" depressurizes the pair of first grip members 210 and 220 of the second linear member moving device 10".

In this case, after the first linear member moving device 10' performs the operation of gripping and moving the linear member 1 in one direction of the extension directions of the linear member 1, the second linear member moving device 10" may then perform the operation of gripping and moving the linear member 1 in one direction of the extension directions of the linear member 1.

In this case, the first linear member moving device 10' and the second linear member moving device 10" may each continuously move the linear member 1 in the extension direction of the linear member 1 with a time difference. As a result, the linear member 1 may be quickly moved in the extension direction of the linear member 1.

In addition, the linear member moving system 5 according to the present embodiment may further include a control unit (not illustrated) for controlling the driving timing of the first linear member moving device 10' and the second linear member moving device 10".

The control unit may detect whether the first linear member moving device 10' has completed moving, for example, through a sensor (for example, a light sensor, an encoder, a stroke sensor, or the like), or determine the operation sequence through built-in program logic or a mutual communication signal.

Through this, after the first linear member moving device 10' has completed moving of the linear member 1, the second linear member moving device 10" operates continuously, so that the two moving devices may efficiently move the linear member 1 in the extension direction.

Such a control method may be implemented hardware-based or software-based, and the control unit 700 may be a central controller or a local controller installed in each mobile device.

Meanwhile, the first linear member moving device 10' and the second linear member moving device 10" constituting the linear member moving system 5 according to one embodiment of the present disclosure may each be equipped with the first driving module 400' illustrated in FIG. 20. In this case as well, the first driving module 400' of the first linear member moving device 10' and the first driving module 400' of the second linear member moving device 10" may provide driving force to continuously move the linear member 1 in the extension direction of the linear member 1 with a time difference.

Although the embodiments of the present disclosure have been described, the spirit of the present disclosure is not limited to the embodiments presented in the present specification, and those skilled in the art who understand the spirit of the present disclosure will be able to easily propose other embodiments by adding, changing, deleting, or adding components within the scope of the same spirit, but this will also be considered to fall within the spirit of the present disclosure.

## Claims

1. A linear member moving device that moves a linear member in an extension direction of the linear member, the linear member moving device comprising:
a main body;
a pair of first grip members that is disposed to face each other with the linear member interposed therebetween and operatively supported by the main body to grip or release the grip of the linear member;
a pair of second grip members that is disposed to face each other with the pair of first grip members interposed therebetween and that pressurizes or depressurizes the pair of first grip members so that the pair of first grip members grips or releases the grip of the linear member and operatively supported by the main body to move in the extension direction of the linear member;
a first driving module that provides a driving force for the pair of second grip members to pressurize or depressurize the pair of first grip members and move in the extension direction of the linear member; and
a second driving module that provides a driving force for the pair of first grip members to rotate about a central axis in the extension direction of the linear member.

2. The linear member moving device of claim 1, wherein the pair of second grip members is movable in the extension direction of the linear member together with the linear member and the pair of first grip members in a state of pressurizing the pair of first grip members, and
the pair of first grip members is rotatable about the central axis in the extension direction of the linear member together with the linear member in a state of being pressurizing by the pair of second grip members,
wherein the pair of second grip members is movable in the extension direction of the linear member in a state where the pair of first grip members is depressurized, and
the pair of first grip members is rotatable about the central axis in the extension direction of the linear member in a state of being depressurized by the pair of second grip members.

3. The linear member moving device of claim 1, wherein the first driving module comprises
a gripper that operates the pair of second grip members to pressurize or depressurize the pair of first grip members, and
an actuator that provides a driving force for moving the gripper in the extension direction of the linear member, and
the gripper comprises
a pair of pressing members that is respectively coupled with the pair of second grip members, and
a gripper actuator that provides a driving force for the pair of second grip members to pressurize or depressurize the pair of first grip members to the pair of pressing members.

4. The linear member moving device of claim 1, wherein the first driving module comprises
a pair of pressing members that is coupled with the pair of second grip member,
a driving motor that provides a driving force for operating the pair of pressing members, and
a pair of link units that operably connects the driving motor and the pair of pressing members, and moves the pair of pressing members along a first trajectory moving in one direction of the extension direction of the linear member and a second trajectory moving away from and toward the linear member while moving in the other direction of the extension direction of the linear member, and
the linear member is gripped when the pair of pressing members moves along the first trajectory, and the grip of the linear member is released when the pair of pressing members move along the second trajectory.

5. The linear member moving device of claim 4, wherein each link unit comprises
a driving link that has one end portion receiving the driving force of the driving motor and rotates about a third direction that is perpendicular to both a first direction, which is the extension direction of the linear member, and a second direction, which is the direction in which the pair of second grip members approaches and moves away from each other,
a connecting link that has one end portion pivotally connected to the other end portion of the driving link and the other end portion coupled to each of the pressing members, and
an auxiliary link that has one end portion pivotally connected to one side of the main body and the other end portion pivotally coupled to a central region of the connecting link, and
the driving links of each link unit receive the driving force of the driving motor and rotate in directions opposite to each other.

6. The linear member moving device of claim 5, wherein the first driving module further comprises a pair of link drive gears that is axial-coupled to rotate together with one end portion of the driving link of each link unit,
the pair of link drive gears is mutually meshed and rotated in directions opposite to each other, and
one of the pair of link drive gears is operably connected to the driving motor to provide a driving force.

7. The linear member moving device of claim 1, wherein the second driving module comprises
a rotating member that is disposed to be spaced apart from the pair of first grip members in the extension direction of the linear member and is supported by the main body so as to rotate about the central axis in the extension direction of the linear member,
a motor that is operably connected to the rotating member to rotate the rotating member, and
a connecting member that interconnects the rotating member and the pair of first grip members, with at least one connecting member provided for each of the first grip members.

8. The linear member moving device of claim 7, wherein the connecting member has a bar shape extending in the extension direction of the linear member, and
a connecting member penetration hole through which the connecting member penetrates is formed in each of the first grip members,
wherein the connecting member guides each of the first grip members when each of the first grip members moves in the extension direction of the linear member.

9. The linear member moving device of claim 7, wherein the connecting member has a bar shape extending in the extension direction of the linear member, and
a connecting member penetration hole through which the connecting member penetrates is formed in each of the first grip members,
wherein the connecting member guides each of the first grip members when each of the first grip members moves in the extension direction of the linear member,
wherein the connecting member penetration hole extends in a direction in which the pair of first grip members comes closer or moves away from each other, and
the connecting member guides the pair of first grip members when the pair of first grip members performs a gripping or grip-releasing operation.

10. The linear member moving device of claim 1, wherein each of the pair of first grip members is formed so that at least a part of one surface facing the linear member is formed as a plane, and at least a part of the other surface located on a side opposite to the one surface is formed as a curved surface, and
an elastic body is disposed between the pair of first grip members to provide an elastic force in a direction in which the pair of first grip members moves away from each other.

11. The linear member moving device of claim 1, wherein each of the pair of second grip members comprises
a second grip member body spaced apart from the linear member in a direction perpendicular to the extension direction of the linear member, and
at least one rolling member coupled to the second grip member body so as to make rolling motion by contacting the pair of first grip members when the pair of first grip members rotates in a state of gripping the linear member.

12. The linear member moving device of claim 1, further comprising a push unit that pushes the pair of first grip members in a movement direction of the second grip member when the pair of second grip members moves in the extension direction of the linear member in a state of completely depressurizing the pair of first grip members.

13. The linear member moving device of claim 12, wherein the push unit is formed to move in the extension direction of the linear member by the driving force of the first driving module moving the pair of second grip members in the extension direction of the linear membe,.
wherein the push unit is supported by a part of the first driving module that moves in the extension direction of the linear member together with the pair of second grip member.

14. The linear member moving device of claim 12, wherein the push unit comprises a pair of push members spaced apart in the extension direction of the linear member with the pair of first grip members interposed therebetween,
wherein each push member comprises,
a rotary plate having a linear member penetration hole formed through which the linear member penetrates and disposed to face any one of both sides of the pair of first grip members in the extension direction of the linear member, and
a push member body that supports the rotary plate so as to be rotatable about the linear member penetration hole,
wherein the linear member moving device further comprises a force sensor interposed between both sides of the pair of first grip members in the extension direction of the linear member and the pair of push members.

15. A linear member moving system comprising a pair of linear member moving devices according to claim 1,
wherein the pair of linear member moving devices comprises a first linear member moving device and a second linear member moving device, and
when a first driving module of the first linear member moving device provides a driving force for moving in one direction of extension directions of the linear member in a state where a pair of second grip members of the first linear member moving device pressurizes a pair of first grip members of the first linear member moving device,
the first driving module of the second linear member moving device provides a driving force for moving in the other direction of the extension directions of the linear member in a state where a pair of second grip members of the second linear member moving device depressurizes a pair of first grip members of the second linear member moving device.
